# FASCICULE DE BREVET EUROPEEN

(11) **EP 1 589 014 B1**
(45) Date de publication et mention de la délivrance du brevet: **10.10.2007**
(21) Numéro de dépôt: 05290382.0
(22) Date de dépôt: 21.02.2005
(51) Int. Cl.: C07D 405/06, C07D 223/16

(54) **Procédé de synthèse de dérives de la 1,3-dihydro-2H-3-benzazepin-2-one et application à la synthèse de l'ivabradine et des sels d'addition à un acide pharmaceutiquement acceptable**
Verfahren zur Herstellung von 1,3-Dihydro-2H-3-Benzazepin-2-on und Anwendung zur Herstellung von Ivabradine und ihren pharmazeutischen annehmbaren Salzen
Process for the preparation of 1,3-dihydro-2H-3-benzazepin-2-one and application to the preparation of ivabradine and their pharmaceutically acceptable salts

(30) Priorité: 13.04.2004 FR 0403829
(43) Date de publication de la demande: 26.10.2005
(73) Titulaire: Les Laboratoires Servier, 92415 Courbevoie Cedex (FR)
(72) Inventeur: Lerestif, Jean-Michel, 76190 Yvetot (FR); Lecouve, Jean-Pierre, 76600 Le Havre (FR); Brigot, Daniel, 76190 Sainte-Marie-des-Champs (FR)

(56) Documents cités:
- EP-A- 0 065 229
- EP-A- 0 109 636
- EP-A- 0 161 604
- EP-A- 0 204 349
- EP-A- 0 292 840

## Description

La présente invention concerne un procédé de synthèse de dérivés de la 1,3-dihydro-2*H*-3-benzazépin-2-one, et leur application à la synthèse de l'ivabradine et de ses sels d'addition à un acide pharmaceutiquement acceptable.

Plus spécifiquement, la présente invention concerne un procédé de synthèse des composés de formule (I) : 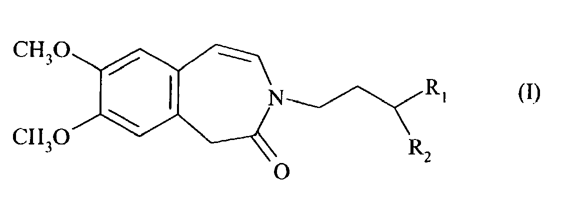 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane.

Les composés de formule (I) obtenus selon le procédé de l'invention sont utiles dans la synthèse de l'ivabradine de formule (II) : 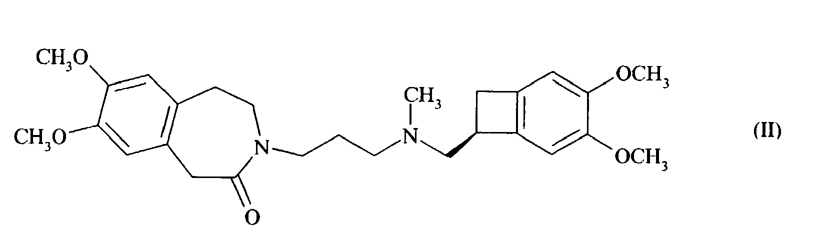 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de ses hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Ce brevet décrit la synthèse du chlorhydrate de l'ivabradine à partir du composé de formule (III) :

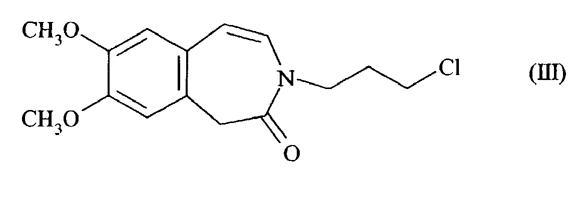

Un accès au composé de formule (III) a été décrit dans la publication J. Med. Chem 1990, Vol. 33 (5), 1496-1504, ainsi que dans le brevet EP 0204349, par alkylation du composé de formule (IV) : 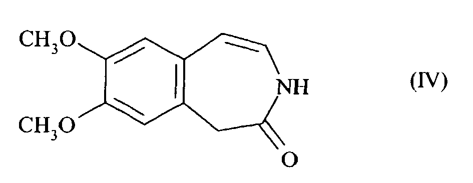 par le 1-bromo-3-chloropropane, dans le diméthylsulfoxyde, en présence de tert-butylate de potassium.

Le brevet EP 0204349 décrit également l'accès au diéthylacétal de formule (Ia) : 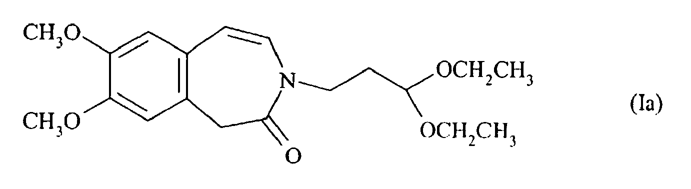 par alkylation du composé de formule (IV) par le diéthylacétal du 3-chloropropionaldéhyde, dans les mêmes conditions (tert-butylate de potassium, diméthylsulfoxyde).

L'utilisation d'une base forte telle que le tert-butylate de potassium correspond aux conditions habituelles requises pour déprotonner une fonction amide.

Cependant, les alcoolates, et notamment le tert-butylate de potassium, présentent l'inconvénient d'être extrêmement hygroscopiques, ce qui complique leur stockage et rend plus délicate leur utilisation à l'échelle industrielle.

Un autre réactif couramment utilisé pour déprotonner une fonction amide est l'hydrure de sodium, utilisé dans la diméthylformamide.

Cependant, le couple hydrure de sodium/diméthylformamide, outre le fait qu'il soit lui aussi très hygroscopique, présente l'inconvénient majeur d'être explosif à des températures relativement basses.

Or, compte-tenu de l'intérêt industriel de l'ivabradine et de ses sels, il était impératif de trouver un procédé performant permettant notamment d'accéder au dérivé de 1,3-dihydro-2*H*-3-benzazépin-2-one de formule (I) avec un bon rendement, tout en évitant l'utilisation de réactifs hygroscopiques.

Or, la Demanderesse s'est aperçue que la réaction d'alkylation du composé de formule (IV) par un composé bromé pouvait tout aussi bien être effectuée en présence de soude en solution aqueuse, d'un carbonate de sodium ou de potassium dont la manipulation est beaucoup plus aisée, en particulier à l'échelle industrielle.

Ce fait est surprenant, car il va à l'encontre de ce qui est habituellement observé pour les lactames, puisque leur déprotonnation requiert habituellement l'utilisation d'une base plus forte.

Plus spécifiquement, la présente invention concerne un procédé de synthèse des composés de formule (I) : 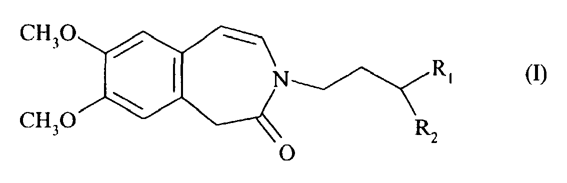 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane,
caractérisé en ce que l'on soumet le composé de formule (IV) : 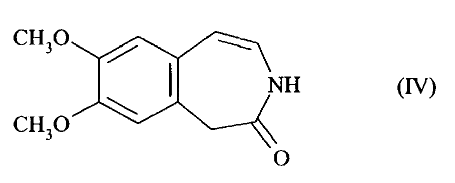 à une réaction d'alkylation par le composé de formule (V) : 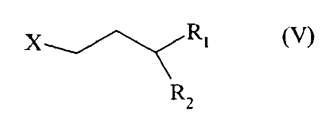 dans laquelle R₁ et R₂ sont tels que définis précédemment, et X représente un groupement partant,
dans la N-méthylpyrrolidone,
en présence d'une base choisie parmi la soude en solution aqueuse et le carbonate de sodium ou de potassium
pour conduire après isolement au composé de formule (I).

Parmi les groupements partants, on peut citer à titre non limitatif les atomes d'halogène, préférentiellement l'atome de brome, et les groupements tosylate, mésylate et triflate.

La quantité de soude est préférentiellement comprise entre 1 et 2 moles par mole de composé de formule (IV).

En présence de soude, la température de réaction est préférentiellement comprise entre 20 et 100°C.

En présence de carbonate de sodium ou de potassium, la température de réaction est préférentiellement supérieure à 80°C.

Dans le procédé selon l'invention, les composés de formule (V) préférentiellement utilisés sont ceux pour lesquels R₁ et R₂ forment ensemble avec l'atome de carbone qui les porte un cycle 1,3-dioxane, 1,3-dioxolane ou 1,3-dioxépane.

Les composés de formule (I) pour lesquels R₁ et R₂ forment ensemble avec l'atome de carbone qui les porte un cycle 1,3-dioxane, 1,3-dioxolane ou 1,3-dioxépane sont des produits nouveaux, utiles comme intermédiaires de synthèse dans l'industrie chimique ou pharmaceutique, et font à ce titre partie intégrante de la présente invention.

Les composés de formule (I) obtenus selon le procédé de la présente invention sont particulièrement utiles comme intermédiaires de synthèse dans la synthèse de l'ivabradine et de ses sels d'addition à un acide pharmaceutiquement acceptable.

A titre d'exemple, l'hydrogénation catalytique d'un composé de formule (I) conduit au composé hydrogéné correspondant de formule (VII) : 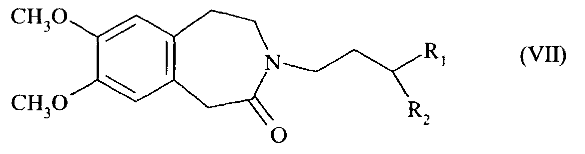 dans laquelle R₁ et R₂ sont tels que définis dans la formule (I),
dont la déprotection du diacétal conduit à l'aldéhyde de formule (VIII) : 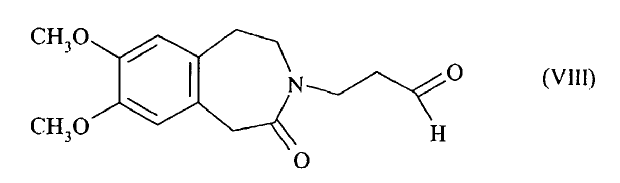 qui est mis en réaction avec la (7S)-3,4-diméthoxybicyclo[4.2.0]octa-1,3,5-trién-7-yl]-N-méthylméthanamine dans des conditions d'amination réductrice, pour conduire à l'ivabradine.

Les exemples ci-dessous illustrent l'invention.

### EXEMPLE 1 : 3-[2-(1,3-Dioxolan-2-yl)-éthyl]-7,8-diméthoxy-1,3-dihydro-2H-3-benzazépin-2-one - mode opératoire en présence de soude

Dans un réacteur, charger 100 g de 7,8-diméthoxy-1,3-dihydro-2*H*-3-benzazépin-2-one, 200 ml de N-méthylpyrrolidone et 115 g de 2-(2-bromoéthyl)-1,3-dioxolane puis amener la température du milieu réactionnel à 40°C et additionner 71 g de lessive de soude à 30 %, en maintenant la température masse à 40°C. Chauffer ensuite le mélange à 60°C, et agiter pendant 2 h, puis couler 400 ml d'eau à 60°C, refroidir le milieu réactionnel à 20°C , puis, après 1 h, à 2°C.

Filtrer le précipité obtenu, le laver et le sécher.

Le produit du titre est obtenu avec un rendement de 87 %.

### EXEMPLE 2 : 3-[2-(1,3-Dioxolan-2-yl)-éthyl]-7,8-diméthoxy-1,3-dihydro-2H-3-benzazépin-2-one- mode opératoire en présence de carbonate de potassium

Dans un réacteur, charger 100 g de 7,8-diméthoxy-1,3-dihydro-2*H*-3-benzazépin-2-one, 250 ml de N-méthylpyrrolidone et 180,4 g de carbonate de potassium, puis agiter ce mélange pendant 3h à 130°C. Additionner ensuite en trois fois, à 2h d'intervalle et à 130°C, sous forte agitation, le 2-(2-bromoéthyl)-1,3-dioxolane (90,8 g, 40,7 g puis 16,3 g).

Après retour à température ambiante, le produit est isolé par précipitation dans l'eau, glaçage, filtration et séchage.

Le produit du titre est obtenu avec un rendement de 80 %.

## Revendications

1. Procédé de synthèse des composés de formule (I) : 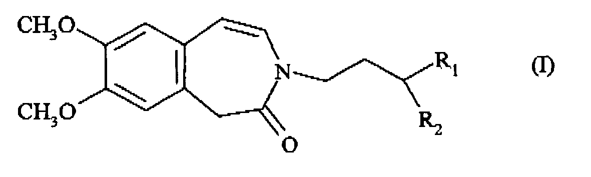 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane,
**caractérisé en ce que** l'on soumet le composé de formule (IV) : 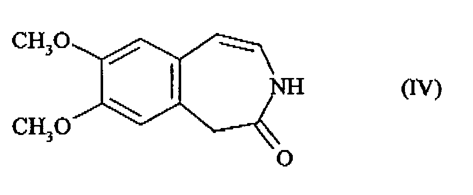 à une réaction d'alkylation par le composé de formule (V) : 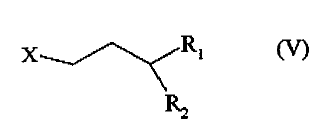 dans laquelle R₁ et R₂ sont tels que définis précédemment, et X représente un groupement partant,
dans la N-méthylpyrrolidone,
en présence d'une base choisie parmi la soude en solution aqueuse et le carbonate de sodium ou de potassium,
pour conduire après isolement au composé de formule (I).

2. Procédé de synthèse selon la revendication 1, **caractérisé en ce qu'**il utilise un réactif de formule (V) pour lequel X représente un atome d'halogène ou un groupement tosylate, mésylate ou triflate.

3. Procédé de synthèse selon la revendication 2, **caractérisé en ce qu'**il utilise un réactif de formule (V) pour lequel X représente un atome de brome.

4. Procédé de synthèse selon l'une quelconque des revendications 1 à 3, **caractérisé en ce qu'**il utilise la soude en solution aqueuse.

5. Procédé de synthèse selon la revendication 4, **caractérisé en ce que** la quantité de soude est comprise entre 1 et 2 moles par mole de composé de formule (IV).

6. Procédé de synthèse selon l'une quelconque des revendications 4 ou 5, **caractérisé en ce que** la température de réaction est comprise entre 20 et 100°C.

7. Procédé de synthèse selon l'une quelconque des revendications 1 à 3, **caractérisé en ce qu'**il utilise le carbonate de sodium ou de potassium.

8. Procédé de synthèse selon la revendication 7, **caractérisé en ce que** la température de réaction est supérieure à 80°C.

9. Procédé de synthèse selon l'une quelconque des revendications 1 à 8, **caractérisé en ce qu'**il utilise comme réactif un composé de formule (V), pour lequel R₁ et R₂ forment ensemble avec l'atome de carbone qui les porte un cycle 1,3-dioxane, 1,3-dioxolane ou 1,3-dioxépane.

10. Composé de formule (I) : 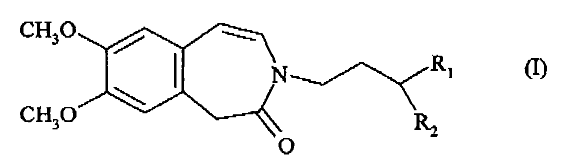 dans laquelle R₁ et R₂ forment ensemble avec l'atome de carbone qui les porte un cycle 1,3-dioxane, 1,3-dioxolane ou 1,3-dioxépane.

11. Procédé de synthèse de l'ivabradine, de ses sels pharmaceutiquement acceptables et de ses hydrates, dans lequel le composé de formule (IV) est transformé en intermédiaire de formule (I) suivant le procédé de la revendication 1, puis l'intermédiaire de formule (I) est transformé en ivabradine.

## Claims

1. Process for the synthesis of compounds of formula (I) : 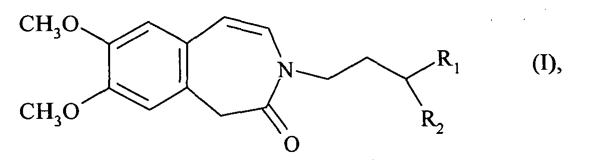 wherein R₁ and R₂, which may be the same or different, each represent a linear or branched (C₁-C₈)alkoxy group or form, together with the carbon atom carrying them, a 1,3-dioxane, 1,3-dioxolane or 1,3-dioxepane ring,
**characterised in that** the compound of formula (IV) : 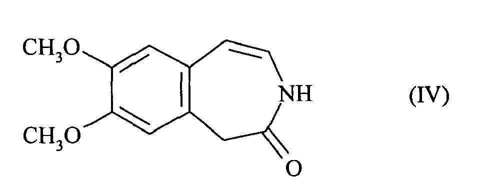 is subjected to an alkylation reaction using the compound of formula (V) : 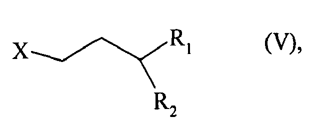 wherein R₁ and R₂ are as defined hereinbefore and X represents a leaving group,
in N-methylpyrrolidone,
in the presence of a base selected from sodium hydroxide in aqueous solution, sodium carbonate and potassium carbonate,
to yield, after isolation, the compound of formula (I).

2. Synthesis process according to claim 1, **characterised in that** there is used a reagent of formula (V) wherein X represents a halogen atom or a tosylate, mesylate or triflate group.

3. Synthesis process according to claim 2, **characterised in that** there is used a reagent of formula (V) wherein X represents a bromine atom.

4. Synthesis process according to any one of claims 1 to 3, **characterised in that** there is used sodium hydroxide in aqueous solution.

5. Synthesis process according to claim 4, **characterised in that** the amount of sodium hydroxide is from 1 to 2 mol per mol of compound of formula (IV).

6. Synthesis process according to either claim 4 or claim 5, **characterised in that** the reaction temperature is from 20 to 100°C.

7. Synthesis process according to any one of claims 1 to 3, **characterised in that** there is used sodium carbonate or potassium carbonate.

8. Synthesis process according to claim 7, **characterised in that** the reaction temperature is higher than 80°C.

9. Synthesis process according to any one of claims 1 to 8, **characterised in that** as reagent there is used a compound of formula (V) wherein R₁ and R₂ form, together with the carbon atom carrying them, a 1,3-dioxane, 1,3-dioxolane or 1,3-dioxepane ring.

10. Compound of formula (I) : 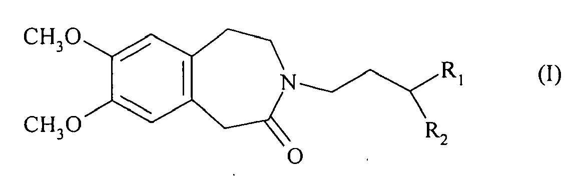 wherein R₁ and R₂ form, together with the carbon atom carrying them, a 1,3-dioxane, 1,3-dioxolane or 1,3-dioxepane ring.

11. Process for the synthesis of ivabradine, pharmaceutically acceptable salts thereof, and hydrates thereof, wherein the compound of formula (IV) is converted into the intermediate of formula (I) in accordance with the process of claim 1 and then the intermediate of formula (I) is converted into ivabradine.

## Patentansprüche

1. Verfahren zur Synthese der Verbindungen der Formel (I): 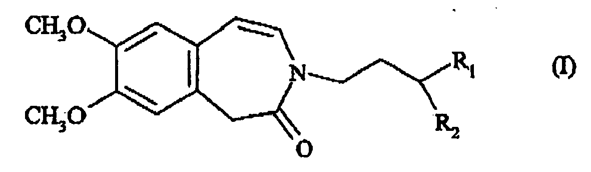 in der R₁ und R₂, die gleichartig oder verschieden sind, jeweils eine geradkettige oder verzweigte (C₁-C₈)-Alkoxygruppe bedeuten oder gemeinsam mit dem sie tragenden Kohlenstoffatom einen 1,3-Dioxan-, 1,3-Dioxolan- oder 1,3-Dioxepan-Ring bilden,
**dadurch gekennzeichnet, daß** man die Verbindung der Formel (IV): 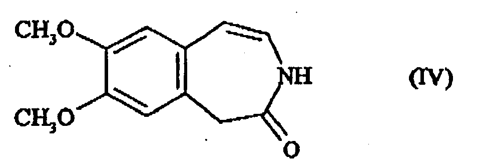 einer Alkylierungsreaktion mit der Verbindung der Formel (V): 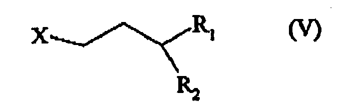 in der R₁ und R₂ die oben angegebenen Bedeutungen besitzen und X eine austretende Gruppe bedeutet,
in N-Methylpyrrolidon
in Gegenwart einer Base ausgewählt aus Natriumhydroxid in wäßriger Lösung und Natriumcarbonat oder Kaliumcarbonat unterwirft,
so daß man nach der Isolierung die Verbindung der Formel (I) erhält.

2. Syntheseverfahren nach Anspruch 1, **dadurch gekennzeichnet, daß** man ein Reagens der Formel (V) verwendet, in der X einHalogenatom oder eine Tosylat-, Mesylat- oder Triflat-gruppe bedeutet.

3. Syntheseverfahren nach Anspruch 2, **dadurch gekennzeichnet, daß** man ein Reagens der Formel (V) verwendet, in der X ein Bromatom bedeutet.

4. Syntheseverfahren nach einem der Ansprüche 1 bis 3, **dadurch gekennzeichnet, daß** man Natriumhydroxid in wäßriger Lösung verwendet.

5. Syntheseverfahren nach Anspruch 4, **dadurch gekennzeichnet, daß** die Menge des Natriumhydroxids zwischen 1 und 2 Mol pro Mol der Verbindung der Formel (IV) beträgt.

6. Syntheseverfahren nach einem der Ansprüche 4 oder 5, **dadurch gekennzeichnet, daß** die Reaktionstemperatur zwischen 20 und 100 °C liegt.

7. Syntheseverfahren nach einem der Ansprüche 1 bis 3, **dadurch gekennzeichnet, daß** man Natriumcarbonat oder Kaliumcarbonat verwendet.

8. Syntheseverfahren nach Anspruch 7, **dadurch gekennzeichnet, daß** die Reaktionstemperatur oberhalb 80 °C liegt.

9. Syntheseverfahren nach einem der Ansprüche 1 bis 8, **dadurch gekennzeichnet, daß** man als Reagens eine Verbindung der Formel (V) verwendet, in der R₁ und R₂ gemeinsam mit dem sie tragenden Kohlenstoffatom einen 1,3-Dioxan-, 1,3-Dioxolan- oder 1,3-Dioxepan-Ring bilden.

10. Verbindung der Formel (I): 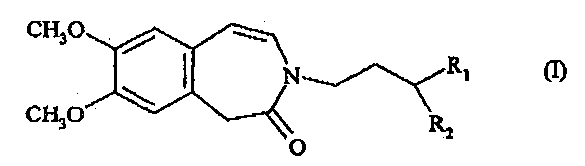 in der R₁ und R₂ gemeinsam mit dem sie tragenden Kohlenstoffatom einen 1,3-Dioxan-, 1,3-Dioxolan- oder 1,3-Dioxepan-Ring bilden.

11. Verfahren zur Synthese von Ivabradin, von seinen pharmazeutisch annehmbaren Salzen und seinen Hydraten, gemäß dem man die Verbindung der Formel (IV) nach dem Verfahren des Anspruchs 1 in das Zwischenprodukt der Formel (I) umwandelt, welches Zwischenprodukt der Formel (I) in Ivabradin umgewandelt wird.
